# EUROPÄISCHE PATENTSCHRIFT

(11) **EP 1 318 137 B1**
(45) Veröffentlichungstag und Bekanntmachung des Hinweises auf die Patenterteilung: **03.05.2006**
(21) Anmeldenummer: 02026361.2
(22) Anmeldetag: 25.11.2002
(51) Int. Cl.: C07C 67/303, C11B 9/00

(54) **Verfahren zur Herstellung von Cyclohexyloxyessigsaürealkylestern**
Process for the preparation of cyclohexyloxyacetic acid alkyl esters
Procédé de préparation d'esters d'alkyle de l'acide cyclohexyloxyacétique

(30) Priorität: 06.12.2001 DE 10160008
(43) Veröffentlichungstag der Anmeldung: 11.06.2003
(73) Patentinhaber: Symrise GmbH & Co. KG, 37603 Holzminden (DE)
(72) Erfinder: Kuhn, Walter, Dr,, 37603 Holzminden (DE); Funk, Hans-Ulrich, 37697 Lauenförde (DE); Senft, Gerhard, 37603 Holzminden (DE)
(74) Vertreter: Eisenführ, Speiser & Partner

(56) Entgegenhaltungen:
- EP-A- 1 078 912
- WO-A-99/18926
- US-A- 3 780 087
- US-A- 3 806 539
- US-A- 4 504 412
- OGAWA K ET AL.: "Studies on Hypolipidemic Agents. III. Synthesis and Esterase-Inhibitory Activity of omega-Cycloalkyl-2-oxyalkyl Arenesulfonates" CHEMICAL AND PHARMACEUTICAL BULLETIN, Bd. 35, Nr. 6, 1987, Seiten 2426-2436, XP001121269

## Beschreibung

Die Erfindung betrifft ein Verfahren zur Herstellung von Cyclohexyloxyessigsäurealkylestern durch Hydrierung von Phenoxyessigsäurealkylestern.

Cyclohexyloxyessigsäurealkylester können als Ausgangsmaterialien zur Herstellung von Riechstoffen verwendet werden. So findet Cyclohexyloxyessigsäuremethylester als Ausgangsmaterial zur Herstellung von Cyclohexyloxyessigsäureallylester Verwendung, wie beispielhaft beschrieben in Perfumer & Flavorist, Vol. 25, Januar-Februar 2000, 19.

Cyclohexyloxyessigsäureallylester ist eine farblose Flüssigkeit mit fruchtigem, ananasartigem Geruch und ist unter dem Namen Isoananat® im Handel (Hersteller: Haarmann & Reimer GmbH).

In Bull. Soc. Chim. Fr. 1928, 4, 903 wird die Herstellung von Cyclohexyloxyessigsäuremethylester durch Hydrolyse von Cyclohexyloxyacetonitril mittels methanolischer Chlorwasserstoff-Lösung ohne Ausbeuteangabe beschrieben.

In Chem. Ber. 1960, 93, 1129 wird Cyclohexyloxyessigsäureethylester durch Umsetzung von Cyclohexanol und Diazoessigsäureethylester mit 26 % Ausbeute hergestellt.

US-A- 3780 087 und US-A- 380 653 betreffen die Herstellung von cyclohexyloxyessigäurealkylestern.

Die erwähnten Verfahren sind auf Grund der nur aufwendig herstellbaren Ausgangsstoffe sowie bezüglich der Ausbeute ungenügend.

Aufgabe der vorliegenden Erfindung war es, ein Verfahren zu finden, das die Herstellung von Cyclohexyloxyessigsäurealkylestem in guter Ausbeute ermöglicht.

Die Erfindung betrifft ein Verfahren zur Herstellung von Cyclohexyloxyessigsäurealkylestern durch Hydrierung von Phenoxyessigsäurealkylestern in Gegenwart eines Katalysators enthaltend mindestens ein Metall der 8. Nebengruppe in elementarer Form, wobei der Katalysator Palladium enthält.

Das erfindungsgemäße Verfahren kann durch die folgende Reaktionsgleichung dargestellt werden:

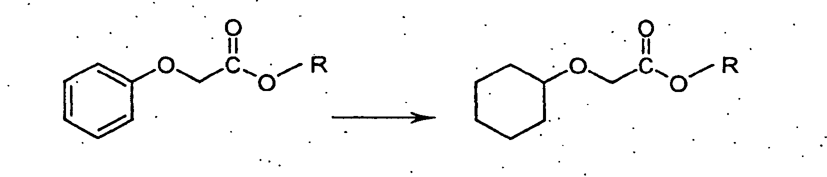

Geeignete Alkylreste R enthalten 1 bis 12 Kohlenstoffatome. Bevorzugte Alkylreste R sind uriverzweigte, verzweigte oder cyclische C₁-C₈-Alkylreste, besonders bevorzugt ist R = Methyl, Ethyl, Propyl, Isopropyl, Butyl, Isobutyl, tertiär Butyl, Pentyl, Isopentyl, Hexyl, Isohexyl, Heptyl, Isoheptyl, Octyl, Isooctyl, Cyclopentyl, Cyclohexyl, Cycloheptyl, Methylcyclopentyl, Methylcyclohexyl, Methylcycloheptyl, Dimethylcyclopentyl, Dimethylcyclohexyl, Ethylcyclopentyl und Ethylcyclohexyl, ganz besonders bevorzugt ist R = Methyl, Ethyl, Propyl, Isopropyl, Butyl, Isobutyl und tertiär Butyl.

Phenoxyessigsäurealkylester sind einfach zugänglich durch Reaktion von Phenol und Chloressigsäurealkylester, wie beispielsweise beschrieben in DE-A 19755598.

Erfindungsgemäß werden unter Katalysatoren Erzeugnisse verstanden, die in dem erfindungsgemäßen Verfahren in der Hydrierung katalytisch wirken.

Die erfindungsgemäßen Katalysatoren enthalten mindestens ein Metall der 8. Nebengruppe in elementarer, metallischer Form.

Diese Metalle können beispielsweise in fein verteilter Form, aufgebracht auf Träger oder zusammen mit anderen Metallen eingesetzt werden (z.B. Mischungen, Legierungen). Die Katalysatoren können Dotierungen mit einem oder mehreren beliebigen Metallen enthalten.

Ein erfindungsgemäß einzusetzenden Katalysator enthält Palladium.

Die erfindungsgemäß einzusetzenden Metalle können auf organische oder anorganische Trägermaterialien aufgebracht sein. Die Katalysatoren können ein Trägermaterial oder Mischungen von Trägermaterialien enthalten. Als vorteilhafte Trägermaterialien seien genannt: Aktivkohle, Kohle, Aluminiumoxide, Metalloxide, Kieselgele, Zeolithe, Tone, Tongranulate, amorphe Aluminiumsilicate und sonstige anorganische Träger. Ein bevorzugtes Trägermaterial ist Aktivkohle

Ein ganz besonders bevorzugter Katalysator ist Palladium auf Aktivkohle.

In dem erfindungsgemäßen Verfahren werden Katalysatormengen eingesetzt, bei denen das Gewichtsverhältnis von Metallmenge zu Phenoxyessigsäurealkylester im Bereich von 1: 100 bis 1 : 10 000 liegt, bevorzugt im Bereich von 1 : 500 bis 1 : 5 000, insbesondere bevorzugt im Bereich von 1 : 1 000 bis 1 : 3 000.

Die Metallmenge bezieht sich dabei auf den absoluten Gehalt des Metalls der 8. Nebengruppe (Palladium) oder den Gesamtgehalt an Metallen der 8. Nebengruppe, d.h. ohne Trägermaterial und ohne eventuell vorhandenes Wasser oder Verdünnungsmittel.

Werden Trägermaterialien enthaltende Katalysatoren verwendet, kann der Anteil an Metall auf dem Trägermaterial im allgemeinen 0,5 bis 50 Gew.-%, bevorzugt 1 bis 20 Gew.-%, insbesondere bevorzugt 3 bis 10 Gew.-%, bezogen auf den trockenen Katalysator, betragen.

Für das erfindungsgemäße Verfahren kann der Katalysator im trockenen oder feuchten Zustand (Restfeuchte an Wasser) verwendet werden.

Das Verfahren kann kontinuierlich, semi-kontinuierlich und diskontinuierlich durchgeführt werden.

Das erfindungsgemäße Verfahren kann unter Verwendung von Lösungsmitteln oder Lösungsmittelgemischen durchgeführt werden. Geeignet sind beispielsweise Alkohole, wässrige Alkohole, Ether, Ester, aromatische oder gesättigte Kohlenwasserstoffe. Beispielsweise können Lösungsmittel wie Methanol, Ethanol, Isopropanol, n-Propanol, Isobutanol, n-Butanol, sek.-Butanol, Tetrahydrofuran, Dibutylether, Ethylenglykoldimethylether, Ethylacetat, Methylacetat, Pentan, Hexan, Heptan, Octan, Cyclopentan, Cyclohexan, Methylcyclohexan, Cyclooctan, Benzol, Toluol, Ethylbenzol oder Xylole verwendet werden.

Bevorzugt wird das Verfahren lösungsmittelfrei durchgeführt.

Die Hydrierung kann bei Temperaturen im Bereich von 50 bis 200°C durchgeführt werden, bevorzugt im Bereich von 70 bis 160°C, insbesondere bevorzugt bei 90 bis 130°C.

Erfindungsgemäß werden die Hydrierungen mit elementarem Wasserstoff durchgeführt.

Der Wasserstoffdruck liegt geeigneterweise bei 1 bis 100 bar, bevorzugt bei 10 bis 30 bar.

Die Reaktionszeit beträgt bevorzugt 1 bis 100 Stunden, insbesondere bevorzugt 10 bis 50 Stunden, besonders bevorzugt bei 20 bis 40 Stunden.

Das erfindungsgemäße Verfahren wird im Allgemeinen wie folgt durchgeführt:

In einem Druckbehälter mit Rührer werden Phenoxyessigsäurealkylester und der Katalysator und gegebenenfalls ein Lösungsmittel vorgelegt. Es wird bei der gewählten Reaktionstemperatur und Wasserstoffdruck hydriert. Der so erhaltene Cyclohexyloxyessigsäurealkylester wird nach Entfernen des Katalysators durch Filtration, Dekantieren oder Zentrifugation erhalten. Bei Bedarf kann eine Reinigung der Cyclohexyloxyessigsäurealkylester erfolgen, beispielsweise durch Destillation.

Nach dem erfindungsgemäßen Verfahren können Cyclohexyloxyessigsäurealkylester in einer Reinheit von über 99 Gew.-% und einer destillierten Ausbeute von 98 % erhalten werden.

Nach dem erfindungsgemäßen Verfahren können Cyclohexyloxyessigsäurealkylester in hoher Reinheit und in sehr guter Ausbeute hergestellt werden. Dadurch ist das erfindungsgemäße Verfahren besonders in wirtschaftlicher und großtechnischer Hinsicht vorteilhaft.

Die nach dem erfindungsgemäßen Verfahren hergestellten Cyclohexyloxyessigsäurealkylester können zu Cyclohexyloxyessigsäureallylester umgesetzt werden.

Bevorzugt zur Herstellung von Cyclohexyloxyessigsäureallylester sind der Cyclohexyloxyessigsäuremethylester, der Cyclohexyloxyessigsäureethylester, der Cyclohexyloxyessigsäureisopropylester, der Cyclohexyloxyessigsäurepropylester, der Cyclohexyloxyessigsäureisobutylester und der Cyclohexyloxyessigsäurebutylester, besonders bevorzugt sind der Cyclohexyloxyessigsäuremethylester, der Cyclohexyloxyessigsäureethylester und der Cyclohexyloxyessigsäureisopropylester.

Die Cyclohexyloxyessigsäurealkylester weisen Riechstoffeigenschaften auf. Insbesondere die Cyclohexyloxyessigsäurealkylester mit Alkylresten umfassend 1 bis 5 Kohlenstoffatome haben Riechstoffeigenschaften mit fruchtigem, ananasartigem Charakter. Als Riechstoffe bevorzugt sind Cyclohexyloxyessigsäurealkylester mit den Alkylresten R = Methyl, Ethyl, Propyl, Isopropyl, Butyl, Isobutyl, tertiär Butyl, Pentyl und Isopentyl. Besonders bevorzugte Riechstoffe sind die Cyclohexyloxyessigsäurealkylester mit R = Methyl, Propyl, Isopropyl und Isopentyl.

Die geruchliche Beschreibung einiger Cyclohexyloxyessigsäurealkylester im einzelnen:
Cyclohexyloxyessigsäuremethylester:
   frisch, fruchtig, lactonig, galbanumartig, krautig
Cyclohexyloxyessigsäureethylester:
   frisch, fruchtig, grapefruit, orange, galbanumartig, krautig
Cyclohexyloxyessigsäureisopropylester:
   frisch, fruchtig, aldehydig, grün, balsamisch, schokoladig
Cyclohexyloxyessigsäurepropylester:
   frisch, fruchtig, aldehydig, grün
Cyclohexyloxyessigsäureisobutylester:
   frisch, fruchtig, aldehydig, grün, schokoladig
Cyclohexyloxyessigsäurebutylester:
   frisch, aldehydig, grün, ananasartig
Cyclohexyloxyessigsäureisoamylester:
   frisch, fruchtig, aldehydig, grün, schokoladig, ananasartig, galbanumartig
Cyclohexyloxyessigsäureisooctylester:
   krautig, grün

Bei den höhergliedrigen Cyclohexyloxyessigsäurealkylestern kann eine fettige Beinote auftreten.

Folgendes Beispiel erläutert die Erfindung.

### Beispiel 1

In einen Rührautoklaven mit Begasungsrührer wurden 2600 g Phenoxyessigsäuremethylester (GC-Reinheit 99,8 Gew.-%), und 40 g Palladium auf Aktivkohle 5 Gew.-% (feucht, 40 Gew.-% Wassergehalt) vorgelegt. Es wurde 33 Stunden bei 110 bis 120°C und 20 bar Wasserstoffdruck hydriert. Nach Filtration wurden 2650 g Cyclohexyloxyessigsäuremethylester mit einer Reinheit von 99,3 Gew.-% erhalten. Der erhaltene Cyclohexyloxyessigsäuremethylester konnte im Wesentlichen ohne Rückstand bei 120°C Sumpftemperatur und 1 mbar destilliert werden. Die Ausbeute d. Th. betrug 98,4 %.

## Patentansprüche

1. Verfahren zur Herstellung von Cyclohexyloxyessigsäurealkylestem durch Hydrierung von Phenoxyessigsäurealkylestern in Gegenwart eines Katalysators enthaltend mindestens ein Metall der 8. Nebengruppe in elementarer Form, **dadurch gekennzeichnet, dass** der Katalysator Palladium enthält.

2. Verfahren nach Anspruch 1, **dadurch gekennzeichnet, dass** der Katalysator mindestens ein anorganisches Trägermaterial enthält.

3. Verfahren nach mindestens einem der Ansprüche 1 bis 2 **dadurch gekennzeichnet, dass** als Katalysator Palladium auf Aktivkohle eingesetzt wird.

4. Verfahren nach mindestens einem der Ansprüche 1 bis 3, **dadurch gekennzeichnet, dass** das Gewichtsverhältnis von Metallmenge zu Phenoxyessigsäurealkylester im Bereich 1 : 100 bis 1 : 10 000 liegt.

5. Verfahren nach mindestens einem der Ansprüche 1 bis 4, **dadurch gekennzeichnet, dass** die Hydrierung lösemittelfrei durchgeführt wird.

6. Verfahren nach mindestens einem der Ansprüche 1 bis 5, **dadurch gekennzeichnet, dass** der Wasserstoffdruck bei 1 bis 100 bar liegt.

## Claims

1. A process for the production of cyclohexyloxyacetic acid alkyl esters by hydrogenation of phenoxyacetic acid alkyl esters in the presence of a catalyst containing at least one metal of subgroup 8 in elemental form, **characterised in that** the catalyst contains palladium.

2. A process according to claim 1, **characterised in that** the catalyst contains at least one inorganic support material.

3. A process according to at least one of claims 1 to 2, **characterised in that** palladium on activated carbon is used as the catalyst.

4. A process according to at least one of claims 1 to 3, **characterised in that** the weight ratio of the quantity of metal to phenoxyacetic acid alkyl ester is in the range of 1 : 100 to 1 : 10 000.

5. A process according to at least one of claims 1 to 4, **characterised in that** the hydrogenation is performed without solvents.

6. A process according to at least one of claims 1 to 5, **characterised in that** the hydrogen pressure is 1 to 100 bar.

## Revendications

1. Procédé de préparation d'esters d'alkyle de l'acide cyclohexyloxyacétique par hydrogénation des esters d'alkyle de l'acide phénoxyacétique en présence d'un catalyseur contenant au moins un métal du 8^{ème} groupe secondaire sous forme élémentaire, **caractérisé en ce que** le catalyseur contient du palladium.

2. Procédé selon la revendication 1, **caractérisé en ce que** le catalyseur comprend au moins un matériau support inorganique.

3. Procédé selon au moins une des revendications 1 à 2, **caractérisé en ce que** l'on utilise comme catalyseur le palladium sur du charbon actif.

4. Procédé selon au moins une des revendications 1 à 3, **caractérisé en ce que** le rapport en poids de la quantité métallique sur l'ester d'alkyle de l'acide phénoxyacétique se situe dans la plage de 1/100 à 1/10 000.

5. Procédé selon au moins une des revendications 1 à 4, **caractérisé en ce que** l'hydrogénation est réalisée sans solvant.

6. Procédé selon au moins une des revendications 1 à 5, **caractérisé en ce que** la pression d'hydrogène se situe vers 1 à 100 bars.
